# EUROPEAN PATENT SPECIFICATION

(11) **EP 0 760 677 B1**
(45) Date of publication and mention of the grant of the patent: **05.09.2001**
(21) Application number: 95919980.3
(22) Date of filing: 26.05.1995
(51) Int. Cl.: A61K 38/26, C07K 14/605

(54) **A PHARMACEUTICAL PREPARATION COMPRISING GLUCAGON**
EINE GLUCAGON ENTHALTENDE PHARMAZEUTISCHE ZUBEREITUNG
PREPARATION PHARMACEUTIQUE CONTENANT DU GLUCAGON

(30) Priority: 26.05.1994 DK 59094
(43) Date of publication of application: 12.03.1997
(73) Proprietor: NOVO NORDISK A/S, 2880 Bagsvaerd (DK)
(72) Inventor: KORNFELT, Troels, 2830 Virum (DK); RASMUSSEN, Henrik, 0386 Oslo (NO); JENSEN, Flemming, Steen, DK-3450 Alleroed (DK)
(74) Representative: Jorgensen, Dan
(86) International application number: PCT/DK95/00208
(87) International publication number: WO 95/32730

(56) References cited:
- EP-A- 0 303 746
- WO-A-93/12811
- US-A- 5 023 088
- US-A- 5 059 587

## Description

### FIELD OF THE INVENTION

The present invention relates to a stabilized pharmaceutical preparation comprising glucagon.

### BACKGROUND OF THE INVENTION

Human glucagon is a polypeptide hormone secreted by α-cells of the pancreatic islets of Langerhans. It is a single-chain polypeptide consisting of 29 amino acid residues the sequence of which is published inter alia in The Merck Index, 10th Edition (1983), Monograph No. 4307.

Glucagon is used for the treatment of hypoglycemia in diabetics due to its glycogenolytic effect on the liver. Glucagon also exerts a spasmolytic effect on smooth muscles which is used clinically in connection with several imaging procedures, especially radiology.

Glucagon for administration by injection is presently marketed in the form of a lyophilized product which is to be reconstituted using a suitable diluent. The lyophilisate contains lactose as the sole excipient.

In the production of those conventional pharmaceutical preparations comprising glucagon, utmost care must be taken in order to avoid decomposition of the glucagon during preparation, dispensing and lyophilization. Furthermore, glucagon undergoes decomposition during storage of the finished product at room temperature. This significantly limits the shelf-life of the conventional preparations.

Thus, there is a need for a more stable formulation of glucagon which retains its activity for extended periods of time at room temperature. Such stabilized preparations comprising glucagon are desirable for emergency treatment of acute hypoglycemia rendering it possible for diabetic patients to carry a dose of glucagon in their hand bag enabling themselves or another person present to treat an incidence of hypoglycemia immediately.

### BRIEF DESCRIPTION OF THE INVENTION

Surprisingly, it has now been found that a significant improvement of the stability of the glucagon in pharmaceutical compositions comprising this peptide can be achieved by including an ampholyte in the composition.

Thus, in its broadest aspect, the present invention relates to a stabilized pharmaceutical composition comprising glucagon and an ampholyte.

In a preferred embodiment, the invention relates to a pharmaceutical composition for parenteral use.

In another preferred embodiment, the invention relates to a pharmaceutical composition for administration by injection.

In another preferred embodiment, the invention relates to a pharmaceutical composition which is a lyophilisate which has to be dissolved in a prescribed amount of solvent before it is administered by injection.

In another preferred embodiment, the invention relates to a pharmaceutical composition for nasal administration.

### DETAILED DESCRIPTION OF THE INVENTION

The invention relates to a stabilized pharmaceutical preparation comprising glucagon and a stabilizing amount of a pharmaceutically acceptable ampholyte, especially an amino acid or dipeptide or a mixture thereof and optionally an excipient. Such preparations retain the glucagon activity at room temperature, e.g. 25°C, for extended periods of time.

US patent No. 5,059,587 (Yamamoto et al.) discloses powder compositions for nasal administration of physiologically active peptides. Various organic acids, i.a. L-glutamic acid and L-aspartic acid, are suggested as absorption promoters. Optionally, a diluent is included in the compositions and among the suggested diluents are amino acids and polyamino acids. However, no composition comprising an amino acid or a polyamino acid is disclosed.

US patent No. 5,023,088 (Wong et al.) discloses a drug delivery system which can be used for the administration of various drugs including pharmacologically active peptides, e.g. glucagon. Sodium bisulfite and histidine hydrochloride are mentioned as examples of stabilizers which may used in case it is desired to include a stabilizer for the drug in the composition.

WO 93/12811 describes a stabilized pharmaceutical composition comprising growth hormone and asparagine.

A pharmaceutically acceptable ampholyte to be used in accordance with the invention may be selected from the group consisting of amino acids (except histidine) or derivatives thereof such as glycine, N-methylglycine (sarcosine), trimethylglycine hydroxide inner salt (betaine), alanine, β-alanine, valine, leucine, nor-leucine, isoleucine, serine, threonine, aspartic acid, glutamic acid, hydroxyglutamic acid, lysine, hydroxylysine, ornithine, arginine, methionine, asparagine and glutamine; dipeptides such as glycylglycine; pharmaceutically acceptable sulfonic acids or derivatives thereof such as taurine; creatinine, and ethylenediaminetetraacetic acid (EDTA).

An amino acid to be used in accordance with the present invention is preferably a naturally occurring α-amino acid except histidine. Such amino acids may be L- or D-amino acids or glycine or a mixture thereof.

Preferably glycine or glycylglycine or a mixture thereof is used.

A pharmaceutical preparation in lyophilized form according to the invention preferably also comprises an excipient, in order to facilitate on one hand the lyophilization and on the other hand rapid and complete redissolution thereof when the preparation is reconstituted before administration by injection. Usually the solvent used for the redissolution of the lyophilisate is sterile water or sterile water to which a preservative e.g. m-cresol or benzyl alcohol has been added.

An excipient may be selected from disaccharides such as lactose, trehalose, and sucrose, sugar alcohols such as sorbitol or mannitol, polysaccharides such as the polymers commercialized as Dextran® products such as Dextrans® 40, Dextrans® 70 or Dextran® 75, and Ficoll® and polyvalent alcohols such as polyethylene glycol or polyvinyl alcohol or a combination of two or more of these.

The excipient is preferably present in an amount of from 10 to 600 micromoles per mg of glucagon giving an optimum stabilization. The excipient preferred according to the invention is lactose.

In a further aspect of the invention, the pharmaceutical preparation of the invention is in the form of a stabilized solution of glucagon.

In order to obtain the desired stabilization, the stabilizing amino acid or dipeptide may be present in an amount of from 0.01 to 50 micromoles per mg of glucagon. The amount of stabilizing amino acid or dipeptide present per dose of pharmaceutical preparation comprising glucagon is according to the invention from 0.1 to 50 micromoles.

A preferred preparation of the invention comprises glycine or glycylglycine in an amount of about 10 micromoles per mg of glucagon. The amount of glycine or glycylglycine is preferably about 10 micromoles per dose.

The pH value of the preparations according to the invention in the form of a reconstituted solution ready for administration is preferably in the interval from 1 to 7 for example from 2 to 7. Preferably, the pH value is in the interval from 2 to 4, for example from 2.3 to 3.8 and most preferred about 2.8.

The dissolution of the glucagon is preferably carried out at a temperature of from 4 to 8°C.

A most preferred preparation according to the invention comprises glucagon, lactose or mannitol as excipient and glycine or glycylglycine or a mixture thereof as a stabilizing agent. Such a preparation has a buffer effect at a pH value of about 2.8 and results in a minimum rate for the decomposition of glucagon. Thus, the invention enables the formulation of a preparation in which glucagon is stable at room temperature.

The amino acid sequence of human glucagon is identical to the amino acid sequence of porcine and bovine glucagon. Hence, glucagon may be isolated by conventional extraction from porcine or bovine pancreatic glands. In the alternative, glucagon may be prepared by full or partial chemical synthesis or by recombinant techniques, e.g. as disclosed in US patent No. 4,826,763.

The pharmaceutical preparation of the invention may be formulated for administration in any suitable way, e.g. by injection of a solution, by oral administration or by administration to a mucosal membrane, e.g. by nasal administration of a powder or a solution. The pharmaceutical preparation may e.g. be presented in the form of a single dose comprised in a vial or cartridge or any other suitable container.

The amount of the stabilized glucagon composition according to the present invention to be given to a patient in need of such a treatment is identical to the amount which would have to be to be given under identical circumstances of a glucagon composition of the known art.

The invention is explained more detailled in the examples below which illustrate the invention. The examples are not to be considered as limiting the scope of the invention which is defined by the appended claims. The features disclosed in the foregoing description and in the following examples may, either separately or in any combination thereof, be material for realising the invention in diverse forms thereof.

### EXAMPLES

### MATERIALS AND METHODS

Preparation and lyophilization of formulations comprising glucagon was carried out using the following procedure:

The glucagon used was recombinant glucagon prepared in Saccharomyces cerevisiae as disclosed in US patent No. 4,826,763.

10.7 g of lactose and the appropriate amount of the amino acid or dipeptide were dissolved in approximately 75 ml of distilled water and the pH value was adjusted to 2.6 - 3.0 using 1 N hydrochloric acid or sodium hydroxide as necessary. The volume was adjusted to 100 ml and the solution was cooled to 4 - 8°C in a refrigerator. Then, 110 mg of glucagon was dissolved in the solution and the pH value was adjusted to 2.6 - 3.0 using 1 N hydrochloric acid while the temperature of the solution was maintained at 4 - 8°C.

The solution was sterile filtered through a 0.2 *µ*m filter (using a 50 ml syringe) and divided into vials, 1 ml in each.

The solution in the vials was lyophilized for a total of 36 hours, using the following procedure:
1) prefreezing at -45°C for 3 - 5 hours, followed by
2) primary drying from -45°C to 20°C and a pressure of 0.1 hPa for 29 hours, and
3) secondary drying at 20°C (and full vacuum) for 4 hours using a Heto CD8 apparatus.

### EXAMPLE 1

### Preparation of formulations comprising glucagon, lactose and an amino acid or a dipeptide and study of their stability.

In an analogous manner as described above, formulations were prepared from glucagon, lactose and an amino acid or a dipeptide.

As reference was used a formulation comprising glucagon and lactose prepared as described above.

Formulations having the following compositions per vial were prepared:

| Reference: | |
|---|---|
| Glucagon | 1.1 mg |
| Lactose USP/Ph Eur | 107 mg |
| 1N HCl | ad pH 2.8 |

| Test formulations: | |
|---|---|
| Glucagon | 1.1 mg |
| Lactose USP/Ph Eur | 107 mg |
| Ampholyte | 5, 10, or 20 mM |
| HCl/NaOH | ad pH 2.8 |

The following ampholytes were tested, each at three concentration levels, 5, 10 and 20 mM, as given under the heading "Conc. AA (mM)" in tables 1 to 3:

| Formulation | |
|---|---|
| A1 | Glycine |
| A2 | Glycylglycine |
| A3 | Histidine |
| A4 | Aspartic acid |
| A5 | Glutamic acid |
| A6 | Leucine |
| A7 | Alanine |
| A8 | Asparagine |
| A9 | Valine |

Vials containing the test formulations and the reference formulation were incubated at 60°C for a period of up to four weeks. Before the start of the incubation and after one week and four weeks, the contents of one vial from each lot and after two weeks and three weeks, the contents of one vial from most of the lots in the incubator (see tables 1 to 3) were analysed. The degradation of the glucagon, expressed as the percentage of glucagon remaining relative to the amount of glucagon inserted in the formulations, was analysed by reversed phase high performance liquid chromatography (RPC). For each of the ampholytes tested, a column in one of the tables 1 to 3 (Heading: "[name of ampholyte] RPC %") gives the results which can be compared with the results achieved with the reference.

The results achieved with the ampholytes show that a very pronounced stabilization of glucagon is obtained by adding a stabilizing agent according to the invention.

**TABLE 1**

| | | Glycine | Glycylglycine | Histidine | Reference |
|---|---|---|---|---|---|
| Week | Conc. AA (mM) | RPC % | RPC % | RPC % | RPC % |
| 0 | 5 | 98.00 | 97.90 | 97.90 | 97.60 |
| | 10 | 97.90 | 97.90 | 98.00 | |
| | 20 | 97.80 | 97.90 | 97.90 | |
| 1 | 5 | 97.10 | 96.40 | 94.70 | 55.80 |
| | 10 | 92.40 | 97.00 | 97.20 | |
| | 20 | 93.40 | 96.10 | 94.00 | |
| 2 | 5 | 91.60 | 95.20 | 91.20 | 29.30 |
| | 10 | 94.50 | 93.70 | 94.40 | |
| | 20 | 91.60 | 90.80 | 90.90 | |
| 3 | 5 | 94.80 | 95.30 | 88.10 | 25.30 |
| | 10 | 93.20 | 94.30 | 87.60 | |
| | 20 | 91.80 | 93.60 | 94.10 | |
| 4 | 5 | 84.60 | 91.70 | 90.90 | 26.90 |
| | 10 | 93.30 | 87.50 | 93.30 | |
| | 20 | 85.40 | 90.10 | 94.20 | |

**TABLE 2**

| | | Aspartic acid | Glutamic acid | Leucine | Reference |
|---|---|---|---|---|---|
| Week | Conc. AA (mM) | RPC % | RPC % | RPC % | RPC % |
| 0 | 5 | 97.70 | 97.10 | 97.70 | 97.80 |
| | 10 | 97.80 | 97.70 | 97.60 | |
| | 20 | 97.70 | 97.70 | 97.60 | |
| 1 | 5 | 72.70 | 60.30 | 81.00 | 45.80 |
| | 10 | 95.60 | 95.80 | 95.90 | |
| | 20 | 94.80 | 94.90 | 95.40 | |
| 2 | 5 | 65.80 | 48.10 | 74.70 | 33.50 |
| | 10 | 93.30 | 94.10 | 94.20 | |
| | 20 | 91.90 | 93.30 | 93.60 | |
| 4 | 5 | 57.80 | 32.70 | 67.00 | 19.20 |
| | 10 | 88.60 | 90.50 | 91.00 | |
| | 20 | 86.50 | 88.20 | 89.10 | |

**TABLE 3**

| | | Alanine | Asparagine | Valine | Reference |
|---|---|---|---|---|---|
| Week | Conc. AA (mM) | RPC % | RPC % | RPC % | RPC % |
| 0 | 5 | 97.30 | 97.30 | 97.40 | 97.00 |
| | 10 | 97.50 | 97.60 | 97.60 | |
| | 20 | 97.30 | 97.60 | 97.30 | |
| 1 | 5 | 93.60 | 93.90 | 85.90 | 68.10 |
| | 10 | 96.30 | 96.20 | 96.10 | |
| | 20 | 95.90 | 96.00 | 95.80 | |
| 3 | 5 | 90.90 | 91.90 | 80.00 | 44.00 |
| | 10 | 94.30 | 94.40 | 94.00 | |
| | 20 | 93.20 | 93.80 | 92.80 | |
| 4 | 5 | 89.90 | 91.40 | 75.40 | 35.90 |
| | 10 | 93.40 | 93.60 | 93.50 | |
| | 20 | 92.30 | 93.00 | 92.10 | |

## Claims

1. A pharmaceutical preparation comprising a) glucagon, b) a stabilizing amount of a pharmaceutically acceptable ampholyte except histidine and c) a pharmaceutically acceptable excipient which is present in an amount of from 10 to 600 micromoles per mg of glucagon.

2. A pharmaceutical preparation as claimed in claim 1, wherein the ampholyte is selected from the group consisting of glycine, N-methylglycine, glycylglycine, trimethylglycine hydroxide inner salt, alanine, β-alanine, valine, leucine, norleucine, isoleucine, serine, threonine, aspartic acid, glutamic acid, hydroxyglutamic acid, lysine, hydroxylysine, ornithine, arginine, methionine, asparagine, glutamine, taurine, creatinine, and ethylenediaminetetraacetic acid.

3. A pharmaceutical preparation as claimed in claim 2, wherein the ampholyte is a pharmaceutically acceptable, naturally occurring amino acid, except histidine, or a dipeptide or a mixture thereof.

4. A pharmaceutical preparation as claimed in claim 3, wherein the ampholyte is glycine or glycylglycine or a mixture thereof.

5. A pharmaceutical preparation as claimed in claim 1 wherein the excipient is selected from disaccharides such as lactose, trehalose, and sucrose and sugar alcohols such as sorbitol or mannitol, polysaccharides such as the polymers commercialized as Dextran® products and Ficoll®, and polyvalent alcohols such as polyethylene glycol or polyvinyl alcohol or a combination of two or more of these.

6. A pharmaceutical preparation as claimed in claim 5 wherein the excipient is lactose.

7. A pharmaceutical preparation as claimed in any of claims 1-6 wherein the glycine or glycylglycine is present in an amount of from 0.01 to 50 micromoles per mg of glucagon.

8. A pharmaceutical preparation as claimed in claim 7 wherein the glycine or glycylglycine is present in an amount about 10 micromoles per mg of glucagon.

9. A pharmaceutical preparation as claimed in any of claims 1-8 which is a solution with a pH value in the interval from 2 to 7.

10. A pharmaceutical preparation as claimed in claim 9 wherein the pH value is in the interval from 2.3 to 3.8.

11. A pharmaceutical preparation as claimed in claim 10 wherein the pH value is about 2.8.

12. A pharmaceutical preparation as claimed in any of claims 1-9 which is a lyophilisate which on dissolution in the amount of solvent prescribed for administration by injection has a pH value in the interval from 2 to 7, preferably from 2.3 to 3.8, more preferred a pH value of about 2.8.

## Patentansprüche

1. Pharmazeutische Zubereitung, umfassend a) Glucagon, b) eine stabilisierende Menge eines pharmazeutisch akzeptablen Ampholyten, mit Ausnahme von Histidin und c) einen pharmazeutisch akzeptablen Arzneimittelträgerstoff, der in einer Menge im Bereich von 10 bis 600 Mikromol pro mg Glucagon vorliegt.

2. Pharmazeutische Zubereitung wie in Anspruch 1 beansprucht, in der der Ampholyt aus der Gruppe bestehend aus Glycin, N-Methylglycin, Glycylglycin, dem inneren Salz von Trimethylglycinhydroxid, Alanin, β-Alanin, Valin, Leucin, Nor-Leucin, Isoleucin, Serin, Threonin, Asparaginsäure, Glutaminsäure, Hydroxyglutaminsäure, Lysin, Hydroxylysin, Omithin, Arginin, Methionin, Asparagin, Glutamin, Taurin, Creatinin und Ethylendiamintetraessigsäure ausgewählt wurde.

3. Pharmazeutische Zubereitung wie in Anspruch 2 beansprucht, in der der Ampholyt eine pharmazeutisch akzeptable, natürlich vorkommende, Aminosäure mit Ausnahme von Histidin ist, oder ein Dipeptid oder eine Mischung davon.

4. Pharmazeutische Zubereitung wie in Anspruch 3 beansprucht, in der der Ampholyt Glycin oder Glycylglycin oder eine Mischung davon ist.

5. Pharmazeutische Zubereitung wie in Anspruch 1 beansprucht, in der der Arzneimittelträgerstoff ausgewählt wurde aus Disacchariden, wie Lactose, Trehalose und Saccharose, sowie Zuckeralkoholen, wie Sorbitol oder Mannitol, Polysacchariden, wie die kommerziell erhältlichen Polymere, wie Dextran® -Produkte und Ficoll® , und polyvalenten Alkoholen, wie Polyethylenglycol oder Polyvinylalkohol oder eine Kombination von zwei oder mehreren dieser Stoffe.

6. Pharmazeutische Zubereitung wie in Anspruch 5 beansprucht, in der der Arzneimittelträgerstoff Lactose ist.

7. Pharmazeutische Zubereitung wie in einem der Ansprüche 1 bis 6 beansprucht, in der Glycin oder Glycylglycin in einer Menge von 0,01 bis 50 Mikromol pro mg Glucagon vorliegt.

8. Pharmazeutische Zubereitung wie in Anspruch 7 beansprucht, in der Glycin oder Glycylglycin in einer Menge von etwa 10 Mikromol pro mg Glucagon vorliegt.

9. Pharmazeutische Zubereitung wie in einem der Ansprüche 1 bis 8 beansprucht, die eine Lösung mit einem pH-Wert, der im Intervall von 2 bis 7 liegt, ist..

10. Pharmazeutische Zubereitung wie in Anspruch 9 beansprucht, bei der der pH-Wert im Intervall von 2,3 bis 3,8 liegt.

11. Pharmazeutische Zubereitung wie in Anspruch 10 beansprucht, bei der der pH-Wert etwa 2,8 beträgt.

12. Pharmazeutische Zubereitung wie in einem der Ansprüche 1 bis 9 beansprucht, die ein Lyophilisat darstellt, das bei Auflösung in der Lösungsmittelmenge, die für die Verabreichung durch Injektion vorgeschrieben ist, einen pH-Wert aufweist, der im Intervall von 2 bis 7 liegt, vorzugsweise von 2,3 bis 3,8, bevorzugter einen pH-Wert von etwa 2,8.

## Revendications

1. Préparation pharmaceutique comprenant a) du glucagon, b) une quantité à effet stabilisant d'un ampholyte acceptable d'un point de vue pharmaceutique, à l'exception de l'histidine, et c) un excipient acceptable d'un point de vue pharmaceutique, qui est présent en une quantité de 10 à 600 micromoles par mg de glucagon.

2. Préparation pharmaceutique selon la revendication 1, dans laquelle l'ampholyte est choisi dans l'ensemble comprenant la glycine, la N-méthylglycine, la glycylglycine, le sel intérieur hydroxyde de triméthylglycine, l'alanine, la β-alanine, la valine, la leucine, la norleucine, l'isoleucine, la sérine, la thréonine, l'acide aspartique, l'acide glutamique, l'acide hydroxyglutamique, la lysine, l'hydroxylysine, l'ornithine, l'arginine, la méthionine, l'asparagine, la glutamine, la taurine, la créatinine et l'acide éthylènediaminetétraacétique.

3. Préparation pharmaceutique selon la revendication 2, dans laquelle l'ampholyte est un acide aminé naturel acceptable d'un point de vue pharmaceutique, à l'exception de l'histidine, ou un dipeptide, ou un mélange de ces derniers.

4. Préparation pharmaceutique selon la revendication 3, dans laquelle l'ampholyte est la glycine ou la glycylglycine ou un mélange de ces dernières.

5. Préparation pharmaceutique selon la revendication 1, dans laquelle l'excipient est choisi parmi les disaccharides tels que le lactose, le tréhalose et le saccharose, et les alcools de sucre tel que le sorbitol ou le mannitol, les polysaccharides tels que les polymères commercialisés sous le nom de Dextran® et Ficoll®, et les polyalcools tel que le polyéthylèneglycol ou le poly(alcool vinylique), ou une combinaison d'au moins deux d'entre eux.

6. Préparation pharmaceutique selon la revendication 5, dans laquelle l'excipient est le lactose.

7. Préparation pharmaceutique selon l'une quelconque des revendications 1 à 6, dans laquelle la glycine ou la glycylglycine est présente en une quantité de 0,01 à 50 micromoles par mg de glucagon.

8. Préparation pharmaceutique selon la revendication 7, dans laquelle la glycine ou la glycylglycine est présente en une quantité d'environ 10 micromoles par mg de glucagon.

9. Préparation pharmaceutique selon l'une quelconque des revendications 1 à 8, qui est une solution ayant un pH compris entre 2 et 7.

10. Préparation pharmaceutique selon la revendication 9, dans laquelle le pH est compris entre 2,3 et 3,8.

11. Préparation pharmaceutique selon la revendication 10, dans laquelle le pH est d'environ 2,8.

12. Préparation pharmaceutique selon l'une quelconque des revendications 1 à 9, qui est un lyophilisat qui après dissolution dans la quantité de solvant prescrite pour administration par injection a un pH compris entre 2 et 7, de préférence entre 2,3 et 3,8 et plus particulièrement un pH d'environ 2,8.
